# EUROPEAN PATENT APPLICATION

(11) **EP 1 207 393 A1**
(43) Date of publication of application: **22.05.2002**
(21) Application number: 00949884.1
(22) Date of filing: 26.07.2000
(51) Int. Cl.: G01N 33/53, G01N 33/543, G01N 33/577

(54) **METHOD FOR IMMUNOLOGICALLY ANALYZING AND ASSAYING ENVIRONMENTAL POLLUTANT**

(30) Priority: 28.07.1999 JP 21355399
(71) Applicant: Takeda Chemical Industries, Ltd., Osaka-shi, Osaka 540-8645 (JP)
(72) Inventor: GODA, Yasuhiro, Osaka 567-0855 (JP); KOBAYASHI, Ayako, Hyogo 663-8003 (JP); FUKUDA, Katsuji, Ikeda-shi, Osaka 563-0043 (JP)
(74) Representative: Helbing, Jörg, Dr. Dipl.-Chem.
(86) International application number: JP0004957
(87) International publication number: WO0107913

(57) **Abstract**

An environmental contaminant, its degradation product or a mixture thereof can be determined or analyzed in high sensitivity by immunoassay using a complex of an anti-immunoglobulin antibody supported on a carrier and a further antibody against the environmental contaminant or its degradation product bound to said anti-immunoglobulin antibody.

## Description

### Art Field Related

The present invention relates to a method for immunoassay of environmental contaminants or their degradation products and a kit to be used therefor.

### Background Art

Recently, a social problem has been caused by environmental pollution due to environmental contaminants such as surfactant compounds for synthetic detergents and endocrine disruptors. Then, it is necessary to determine and analyze environmental contaminants and their degradation products and to utilize the results for environmental preservation.

Various methods for determination and analysis of such environmental contaminants and their degradation products have been known heretofore in the prior art. For example, for quantitative determination of environmental contaminants and their degradation products in tap water, rivers, swamps, lakes or sewage, there are high-performance liquid chromatography (HPLC), gas chromatography (GC), gas chromatography-high resolution mass spectrometry (GC-HRMS) and high-performance liquid chromatography-high resolution mass spectrometry (HPLC-HRMS), and the like. However, in these known methods, there are such problems that sensitivity of these methods are insufficient for that required for determination of environmental contaminants which are present in a trace amount in specimens, that these methods require extraction with organic solvents, and that they require expensive apparatuses or devices, pretreatment and the operation with great skill. In view of these problems, it is desired to develop a method for determination of environmental contaminants which is highly sensitive and can be readily and quickly operated with high specificity at a low cost.

As one means for solving these problems, there is an enzyme-linked immunosorbent assay (hereinafter sometimes abbreviated to ELISA).

Assay systems and kits utilizing ELISA have been constructed for various different environmental contaminants. For example, WO 94/12536 discloses ELISA for detecting petroleum fuel in an environment and a kit for such ELISA. The kit comprises an enzyme and an antibody, and the measurement can be completed very quickly, usually, within several hours. And, the operation is very simple in comparison with conventional HPLC, GC, GC-HRMS and HPLC-HRMS. Further, a very specific determination can be carried out by using an antibody, in particular, a monoclonal antibody. Moreover, HPLC, GC, GC-HRMS and HPLC-HRMS require very expensive apparatuses and devices and their maintenance is also expensive, while ELISA has no such problem.

Nevertheless, any immunoassay method which can be used for detection and determination of environmental contaminants and their degradation products, in particular, ELISA has not yet been established.

### Objects of the Invention

One object of the present invention is to provide an immunoassay method which can detect and determine environmental contaminants and their degradation products in high sensitivity.

Another object of the present invention is to provide a kit for using the method of the present invention.

These objects as well as other objects and advantages of the present invention will apparent to those skilled in the art from the following description with reference to the accompanying drawings.

### Brief Explanation of Drawings

Fig. 1 is a calibration curve for determination of estradiol obtained in Example 1 hereinafter using a carrier on which anti-mouse IgG antibody + anti-estrogen antibody are immobilized.

Fig. 2 is a calibration curve for determination of estradiol obtained in Example 1 hereinafter using a carrier on which anti-estrogen antibody is immobilized.

Fig. 3 is a calibration curve for determination of bisphenol A (BPA) obtained in Example 2 using a carrier on which anti-mouse IgA antibody + anti-plastic resin component (PRC) antibody are immobilized.

Fig. 4 is a calibration curve for determination of BPA obtained in Example 2 using a carrier on which anti-PRC antibody is immobilized.

### Summary of the Invention

The present inventors have studied to establish a method for immunoassay of environmental contaminants and their degradation products, in particular, a method for detection and determination thereof by ELISA, intensively. As a result, it has been found that an environmental contaminant or its degradation product can be determined in high sensitivity by using a complex of an anti-immunoglobulin antibody (anti-Ig antibody) supported on a carrier and a further antibody against the environmental contaminant or its degradation product bound to said anti-Ig antibody. The present inventors have further studied based on this finding. Thus, the present invention has been completed.

That is, according to the present invention, there is provided:
(1) a kit for immunoassay of an environmental contaminant, its degradation product or a mixture thereof which comprises as an essential constitutional component a complex of an anti-immunoglobulin antibody supported on a carrier and a further antibody against the environmental contaminant or its degradation product bound to said anti-immunoglobulin antibody;
(2) the kit according to the above (1), wherein the environmental contaminant is a surfactant compound for synthetic detergents or an endocrine disruptor;
(3) the kit according to the above (2), wherein the surfactant compound is a compound selected from the group consisting of anionic surfactant compounds and nonionic surfactant compounds;
(4) the kit according to the above (2), wherein the surfactant compound is a compound selected from the group consisting of C₂₋₁₈ alkyl sulfate, C₂₋₁₈ alkylbenzene sulfonate, C₂₋₁₈ alkylnaphthalene sulfonate, naphthalene sulfonate-formaldehyde condensate, polyoxyethylene C₂₋₁₈ alkylphenyl ether, polyoxyethylene C₂₋₁₈ alkyl ether, polyoxyether polystyryl phenyl ether, polyoxyethylene styrylated phenyl ether and salts thereof;
(5) the kit according to the above (2), wherein the endocrine disruptor is a substance selected from the group consisting of female sex hormones, male sex hormones, thyroid hormones, alkylphenol ethoxylates, alkylphenols, plastic resin components, plastic plasticizers and chlorophenols;
(6) the kit according to the above (2), wherein the endocrine disruptor is a substance selected from the group consisting of estrogen, estradiol, estrone, estriol, androgen, testosterone, dehydroepiandrosterone, androstenedione, thyroxine, triiodothyronine, C₁₋₂₀ alkylphenol ethoxylates, C₁₋₂₀ alkylphenols, bisphenol A, 4,4'-ethylidene bisphenol, bis(p-hydorxyphenyl)methane, 2,2'-bis(4-hydroxypehyl)-1-propanol, 2,2'-bis(m-methyl-p-hydroxyphenyl)propanel, 4,4'-bis(p-hydroxyphenyl)pentanoic acid, 4,4'-diaminodiphenylmethane, 4,4'-dihydroxydiphenyl ether, 4,4'-dihydroxydiphenylsulfone, 4,4'-dichlorodiphenylsulfone, bis[4-(2-hydroxyethoxy)-3,5-dibromophenyl]sulfone, bis[4-(2-hydroxyethoxy)phenyl]sulfone, p,p'-dihydorxybenzophenone, 4-hydroxybiphenol, butyl benzyl phthalate, dibutyl phthalate, dicyclohexyl phthalate, diethyl phthalate, di(2-ethylhexyl) phthalate, diethyl hexyl adipate, dihexyl phthalate, di-n-pentyl phthalate, dipropyl phthalate, and mono-, di-, tri-, tetra- and penta-chlorophenols;
(7) the kit according to the above (1), wherein the anti-immunoglobulin antibody is anti-IgG antibody or a antibody mixture containing it;
(8) a method for immunoassay of an environmental contaminant, its degradation product or a mixture thereof which comprises:
   reacting (a) a specimen, (b) a complex of an anti-immunoglobulin antibody supported on a carrier and a further antibody against the environmental contaminant or its degradation product bound to said anti-immunoglobulin antibody, and (c) the environmental contaminant, its degradation product or a mixture thereof labeled with a labeling agent, and
   measuring the activity of either the labeling agent held on the carrier or the labeling agent not held on the carrier;
(9) the method according to the above (8), wherein the environmental contaminant is a surfactant compound for synthetic detergents or an endocrine disruptor;
(10) the method according to the above (9), wherein the surfactant compound is a compound selected from the group consisting of anionic surfactant compounds and nonionic surfactant compounds;
(11) the method according to the above (9), wherein the surfactant compound is a compound selected from the group consisting of C₂₋₁₈ alkyl sulfate, C₂₋₁₈ alkylbenzene sulfonate, C₂₋₁₈ alkylnaphthalene sulfonate, naphthalene sulfonate-formaldehyde condensate, polyoxyethylene C₂₋₁₈ alkylphenyl ether, polyoxyethylene C₂₋₁₈ alkyl ether, polyoxyether polystyryl phenyl ether, polyoxyethylene styrylated phenyl ether and salts thereof;
(12) the method according to the above (9), wherein the endocrine disruptor is a substance selected from the group consisting of female sex hormones, male sex hormones, thyroid hormones, alkylphenol ethoxylates, alkylphenols, plastic resin components, plastic plasticizers and chlorophenols;
(13) the method according to the above (9), wherein the endocrine disruptor is a substance selected from the group consisting of estrogen, estradiol, estrone, estriol, androgen, testosterone, dehydroepiandrosterone, androstenedione, thyroxine, triiodothyronine, C₁₋₂₀ alkylphenol ethoxylates, C₁₋₂₀ alkylphenols, bisphenol A, 4,4'-ethylidene bisphenol, bis(p-hydorxyphenyl)methane, 2,2'-bis(4-hydroxypehyl)-1-propanol, 2,2'-bis(m-methyl-p-hydroxyphenyl)propanel, 4,4'-bis(p-hydroxyphenyl)pentanoic acid, 4,4'-diaminodiphenylmethane, 4,4'-dihydroxydiphenyl ether, 4,4'-dihydroxydiphenylsulfone, 4,4'-dichlorodiphenylsulfone, bis[4-(2-hydroxyethoxy)-3,5-diburomophenyl]sulfone, bis[4-(2-hydroxyethoxy)phenyl]sulfone, p,p'-dihydorxybenzophenone, 4-hydroxybiphenol, butyl benzyl phthalate, dibutyl phthalate, dicyclohexyl phthalate, diethyl phthalate, di(2-ethylhexyl) phthalate, diethyl hexyl adipate, dihexyl phthalate, di-n-pentyl phthalate, dipropyl phthalate, and mono-, di-, tri-, tetra- and penta-chlorophenols;
(14) the method according to the above (8), wherein the anti-immunoglobulin antibody is anti-IgG antibody or a antibody mixture containing it;
(15) use of a complex of an anti-immunoglobulin antibody supported on a carrier and a further antibody against the environmental contaminant or its degradation product bound to said anti-immunoglobulin antibody in immunoassay of an environmental contaminant, its degradation product or a mixture thereof; and
(16) use according to the above (15), wherein the anti-immunoglobulin antibody is anti-IgG antibody or a antibody mixture containing it.

### Detailed Explanation of the Invention

Environmental contaminants to be the subject of the immunoassay of the present invention include any material which pollutes the environment. Examples thereof include surfactant compounds for synthetic detergents, endocrine disruptors and the like.

As surfactant compounds, there are, for example, anionic surfactant compounds, nonionic surfactant compounds and the like.

Examples of anionic surfactant compounds include those selected from the group consisting of alkyl sulfate, alkylbenzene sulfonate (hereinafter, sometimes, abbreviated to LAS), alkylnaphthalene sulfonate, naphthalene sulfonate-formaldehyde condensate and salts thereof (e.g., salts with alkali metals such as sodium, potassium, etc.).

Examples of nonionic surfactant compounds include those selected from the group consisting of polyoxyethylene alkylphenyl ether, polyoxyethylene alkyl ether, polyoxyether polystyryl phenyl ether, polyoxyethylene styrylated phenyl ether and salts thereof (e.g., phosphates and sulfates). The alkyl groups used herein include, for example, those having 2 to 18 carbon atoms.

As endocrine disruptors, there are, for example, female sex hormones, male sex hormones, thyroid hormones, alkylphenol ethoxylates, alkylphenols, plastic resin components, plastic plasticizers, chlorophenols and the like.

Examples of female sex hormones include estrogen, estradiol (E2), estrone (E1), estriol (E3), and the like. Examples of male sex hormones include androgen, testosterone, dehydroepiandrosterone, androstenedione, and the like. Examples of thyroid hormones include thyroxine (T3), triiodothyronine (T4), and the like. In addition, conjugates of these substances (e.g., glucuronide, sulfate conjugate, etc.) which are metabolites in a living body are also included.

As alkylphenol ethoxylates (APE), for example, there are compounds represented by the formula (1): 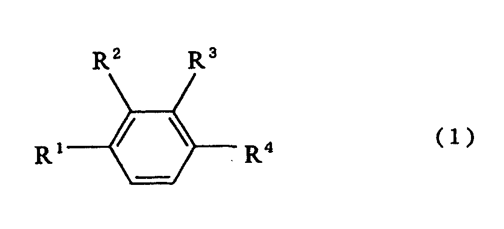 wherein R¹, R² and R³ are the same or different and each is hydrogen atom or a straight or branched (including sec-, tert-, iso-) alkyl group having 1 to 20, preferably 1 to 12 carbon atoms; R⁴ is (OC₂H₄)ₘOH or (OC₂H₄)ₘCOOH; and m is the average number of ethylene oxide chain of 1 to 70, preferably 1 to 15. Examples thereof include nonylphenol ethoxylate (NPE), e.g., NP2EO (the average number of ethylene oxide chain = 2), NP5EO (the average number of ethylene oxide chain = 5), NP10EO (the average number of ethylene oxide chain = 10), NP10EC (the average number of ethylene oxide chain = 10, the terminal OH → carboxylic acid], octylphenol ethoxylate (OPE), and the like.

As alkylphenols (AP), for example, there are compounds represented by the formula (2): 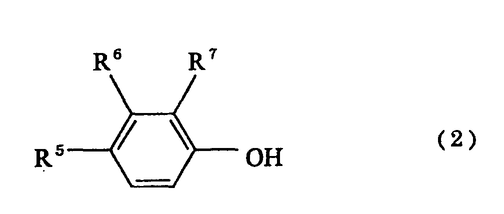 wherein R⁵, R⁶ and R⁷ are the same or different and each is hydrogen atom or a straight or branched (including sec-, tert-, iso-) alkyl group having 1 to 20, preferably 1 to 12 carbon atoms. Examples thereof include 4-dodecylphenol (DP), 4-ethylphenol (EP), heptylphenol (HP), 4-isopentylphenol (IPP), 2-octylphenol (2-OP), 4-nonylphenol (4-NP), 4-octylphenol (4-OP), 4-sec-butylphenol (4-sBP), 4-tert-butylphenol (4-tBP), 4-tert-pentylphenol (4-tPP), 4-tert-octylphenol (4-tOP), and the like.

As plastic resin components (PRC), for example, there are compounds represented by the formula (3): 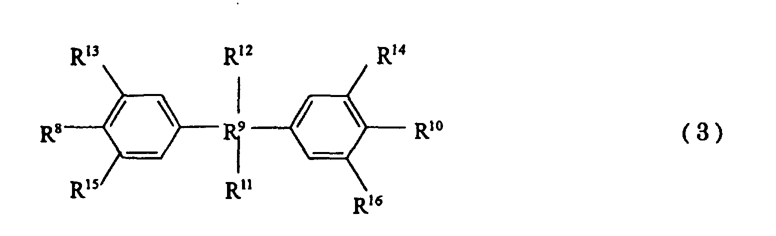 wherein R⁹ is carbon atom, CO or SO₂; R⁸ and R¹⁰ are the same or different and each is hydrogen atom, OH, NH₂ or O(CH₂)₂OH; R¹¹ and R¹² are the same or different and each is absent, hydrogen atom, CH₃, CH₂OH or C₂H₄COOH; and R¹³, R¹⁴, R¹⁵ and R¹⁶ are the same or different and each is hydrogen atom, OH, CH₃, Cl or Br. Examples thereof include bisphenol A (BPA), 4,4'-ethylidene bisphenol (4,4'-EBP), bis(p-hydorxyphenyl)methane (BHPM), 2,2'-bis(4-hydroxypehyl)-1-propanol (2,2'-BHPP), 2,2'-bis(m-methyl-p-hydroxyphenyl)propanel (2,2'-BMHPP), 4,4'-bis(p-hydroxyphenyl)pentanoic acid (4,4'-BHPP), 4,4'-diaminodiphenylmethane (4,4'-DDM), 4,4'-dihydroxydiphenyl ether (4,4'-DDE), 4,4'-dihydroxydiphenylsulfone (4,4'-DOHDS), 4,4'-dichlorodiphenylsulfone (4,4'-DClDS), bis[4-(2-hydroxyethoxy)-3,5-dibromophenyl]sulfone (BHEDBrPS), bis [4-(2-hydroxyethoxy)phenyl]sulfone (BHEPS), p,p'-dihydroxybenzophenone (p,p'-DBP), 4-hydroxybiphenol (HBP), and the like.

As plastic plasticizers (PP), for example, there are compounds of the formula (4): 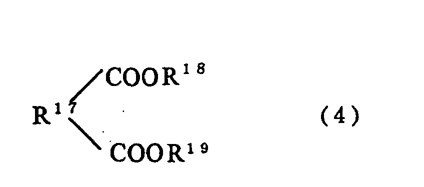 wherein R¹⁷ is o-phenylene or tetramethylene; and R¹⁸ and R¹⁹ are the same or different and each is hydrogen atom, a straight or branched (including sec-, tert-, iso-) alkyl group having 1 to 20, preferably 1 to 12 carbon atoms, benzyl or cyclohexyl. Examples thereof include butyl benzyl phthalate (BBP), dibutyl phthalate (DBP), dicyclohexyl phthalate (DCHP), diethyl phthalate (DEP), di(2-ethylhexyl) phthalate (DEHP), diethyl hexyl adipate (DEHA), dihexyl phthalate (DHP), di-n-pentyl phthalate (DPP), dipropyl phthalate (DPrP), and the like.

As chlorophenols (CP), for example, there are compounds represented by the formula (5): 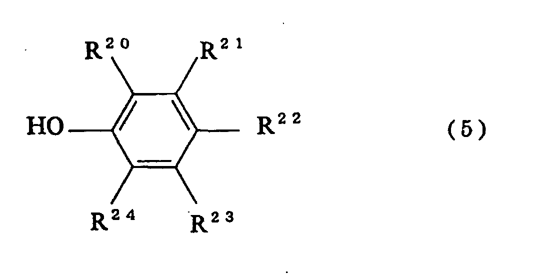 wherein R²⁰, R²¹, R²², R²³ and R²⁴ are the same or different and each is hydrogen atom or Cl. Examples thereof include 2-chlorophenol (2-CP), 3-chlorophenol (3-CP), 4-chlorophenol (4-CP), 2,3-dichlorophenol (2,3-CP), 2,4-dichlorophenol (2,4-CP), 2,5-dichlorophenol (2,5-CP), 2,6-dichlorophenol (2,6-CP), 2,3,4-trichlorophenol (2,3,4-CP), 2,4,5-trichlorophenol (2,4,5-CP), 2,4,6-trichlorophenol (2,4,6-CP), 2,3,4,5-tetrachlorophenol (2,3,4,5-CP), 2,3,4,6-tetrachlorophenol (2,3,4,6-CP), pentachlorophenol (PCP), and the like.

As other materials, there are polychlorodibenzo-p-dioxine (PCDD) whose typical example is 2,3,7,8-tetrachlorodibenzo-p-dioxine (TCDD), polychlorodibenzofuran (PCDF) whose typical example is 2,3,7,8-tetrachlorodibenzofuran (TCDF), polychlorobiphenyl (PCB), benzophenone, benzopyran, chlorobenzene, buromonaphthol, nitrotoluene, tributyltin, various agrochemicals, heavy metals (e.g., Cd, Hg, Pb, etc.), synthesized estrogen (e.g., centchroman, ethinylestradiol, diethylstilbestrol, hexestol, 2-hydroxyestradiol, tamoxifen, laroxyfen, etc.) food, food addivites [e.g., butyl hydroxy anisol (BHA), ecol, enterolactone, phytoestrogen, coumestrol, formononetin, daidzein, biotinin A, gestanin, etc.], and the like.

These environmental contaminants can be appropriately selected according to a particular environmental contaminant or a degradation product thereof to be analyzed.

Examples of the above straight or branched alkyl group having 1 to 20 carbon atoms include methyl, ethyl, propyl, iso-propyl, butyl, iso-butyl, sec-butyl, tert-butyl, n-pentyl, iso-pentyl, neo-pentyl, tert-pentyl, n-hexyl, iso-hexyl, n-heptyl, n-octyl, n-nonyl, n-decyl, undecyl, dodecyl, tridecyl, tetradecyl, pentadecyl, hexadecyl, heptadecyl, octadecyl, nonadecyl, eicosyl, and the like. Examples of the above straight or branched alkyl group having 2 to 18 carbon atoms include those other than methyl and eicosyl.

The anti-Ig antibody to be used in the present invention is an antibody against an immunoglobulin (Ig) which is an antibody against an environmental contaminant or its degradation product.

Preferably, the Ig is that derived from, for example, mammals such as mouse, rat, rabbit, goat, sheep, human, cat, dog, guinea pig, horse, cattle, pig, deer, kangaroo, and the like and poultry such as chicken, turkey, and the like.

As the Ig, there are IgG, IgA, IgM, IgD, IgE, or the like. The Ig may also be a part of an Ig. Examples of a part of Ig include IgG (H+L), IgG (γ chain), IgG (Fab), IgG (Fc), IgG (Fab monomer), IgG (Fd), IgG1, IgG2, IgG3, IgG4, IgG2a, IgG2b, IgG2c, IgA (α chain), IgA1, IgA2, IgM (µ chain), IgD (δ chain), IgE (ε chain), κ light chain, κ (free) chain, λ light chain, λ (free) chain, and the like.

As the anti-Ig antibody to be used in the present invention, for example, there are anti-IgG antibody, anti-IgA antibody, anti-IgM antibody, anti-IgD antibody, anti-IgE antibody, and the like. Among them, anti-IgG antibody is preferred and it may be a mixture of anti-IgG antibody, anti-IgA antibody and anti-IgM antibody.

The Ig which is an antibody against an environmental contaminant or its degradation product, and an antibody against the Ig (i.e., anti-Ig antibody) can be produced by a per se known method, for example, the process disclosed in ENZYME IMMUNOASSAY, pages 46-71 and 85-110 [P. Tijssen, Japanese Version (supervisory translator Eiji Ishikawa), Tokyo Kagaku Dojin (1989)] or its modification.

In these methods, an animal is inoculated with a complex of a derivative of the antigen, i.e., an environmental contaminant or its degradation product and a carrier protein. As the carrier protein to be used, for example, there are bovine serum albumin (hereinafter abbreviated to BSA), ovalbumin (hereinafter abbreviated to OVA), keyhole limpet hemocyanin (hereinafter abbreviated to KLH), bovine thyroglobulin (hereinafter abbreviated to BTG), and the like.

The formation of the complex the derivative of a environmental contaminant or its degradation product and the carrier protein can be carried out by binding a compound represented by the formula (6):

A-R (6)

wherein R is COOH, NH₂ or SH; and A is a group which forms the environmental contaminant or its analogous compound by removal of the group R, to the carrier protein according to a per se known method.

The compound of the formula (6) can also be synthesized chemically by introducing or forming a carboxyl group, amino group or sulfhydryl group in a suitable starting material according to a per se known method.

For example, the compound of the formula (6) wherein R is COOH and A is a group which forms a polyoxyethylene alkylphenyl ether can be produced by subjecting a polyoxyethylene alkylphenyl ether and succinic anhydride to dehydration-condensation (formation of half ester) [Cancer Biochem. Biophys., 7, 175 (1984)].

The compound of the formula (6) wherein R is NH₂ and A is a group which forms a polyoxyethylene alkylphenyl ether can be produced by chlorinating the hydroxyl group of the polyoxyethylene alkylphenyl ether with thionyl chloride [J. Am. Chem. Soc., 60, 2540 (1938)], followed by treatment with ammonia (Organic Functional Group Preparations, Vol. 1, p. 382].

The compound of the formula (6) wherein R is SH and A is a group which forms a polyoxyethylene alkylphenyl ether can be produced by chlorinating hydroxyl group of the polyoxyethylene alkylphenyl ether with thionyl chloride [J. Am. Chem. Soc., 60, 2540 (1938)], followed by reaction with sodium hydrosulfide [J. Am. Chem. Soc., 72, 1843 (1950)].

The carrier to be used may be that normally used in an immunoassay. Examples thereof include microplates (e.g., 96 well microplate, 24 well microplate, 192 well microplate, 384 well microplate, etc.), test tubes (e.g., glass tube, plastic tube, etc.), glass particles, polystyrene partilces, modified polystyrene particles, polyvinyl particles, latex (e.g., polystyrene latex), nitrocellulose membrane, filter paper activated with cyano bromide, filter paper activated with DBM, particulate solid phase (e.g., Sepharose, Sephadex, agarose, cellulose, Sephacryl, etc.), iron-containing polycarbonate membrane, magnet-containing beads, and the like.

The anti-Ig antibody can be supported on the carrier by a per se known method, for example, the above ENZYME IMMUNOASSAY, pages 268-296, AFFINITY CHROMATOGRAPHY HANDBOOK (Amarsham Pharmacia Biotech, published December 20, 1998) and the like.

The other antibody which constitutes the complex of the present invention can be produced by a per se known method. That is, the antibody can be produced by immunizing an animal with the above complex of a derivative of the above environmental contaminant or its degradation product and the carrier protein. The antibody may be a polyclonal antibody obtained by the immunized animal. Alternatively, it may be a monoclonal antibody produced by a hybridoma obtainable by fusing a spleen cell or lymphocyte of the immunized animal with a myeloma cell.

For immunization of an animal, the animal is inoculated with an environmental contaminant or the above-obtained complex. Examples of animals to be inoculated include goat, sheep, rabbit, rat, mouse, guinea pig, chicken and the like. In case that a monoclonal antibody against an environmental contaminant is desired, a mouse is particularly preferred.

The inoculation can be carried out according to a conventional method. For example, a mouse is inoculated with about 1 to 100 µg, preferably about 50 to 100 µg of the immunogen which is emulsified with equal amounts (0.1 ml) of physiological saline and Freund's complete adjuvant or RIBI adjuvant system™ per once at the back or abdomen subcutaneously or intraperitoneally. The mouse receives the inoculation 3 to 6 times at 2 to 3 week intervals. The polyclonal antibody can be collected from the serum of the immunized animal. For obtaining the monoclonal antibody, the immunized animal is further treated as follows.

Among the immunized animals, for example, mice, the individual having a high antibody titer is selected and, 3 to 5 days after the final immunization, the spleen or lymph node is collected. Then, the antibody producing cells contained therein are fused together with myeloma cells.

The fusion can be carried out by a known method and, as a fusion promoting agent, for example, polyethylene glycol (hereinafter abbreviated to PEG) or Sendai virus can be used. PEG is preferred.

As myeloma cells, for example, NS-1, P3U1 or Sp2/O can be used and, in particular, P3U1 is preferred. For example, the preferred proportion of the spleen cells : myeloma cells is 1 : 1 to 10 : 1 and PEG having a molecular weight of about 1,000 to 6,000 is added thereto in concentration of about 10 to 80%. Desirably, the mixture is incubated at about 20 to 37°C, preferably, at about 30 to 37°C for about 3 to 10 minutes.

For screening of the desired hybridoma, various methods can be employed. For example, there is ELISA, wherein a supernatant of a hybridoma culture is added to a microplate on which OVA combined with an environmental contaminant analogue (hapten) has been adsorbed and then an anti-mouse immunoglobulin antibody labeled with horseradish peroxidase (hereinafter abbreviated to HRP) is added thereto to detect the antibody combined to the solid phase of the plate. A hybridoma having positive antibody activity is subjected to cloning immediately. Normally, this can be readily carried out by limiting dilution analysis, or the like.

The antibody titer of cloned hybridomas is determined by the above method and a hybridoma which constantly produces an antibody having a high titer is selected to obtain the desired monoclonal hybridoma.

Examples of the antibody producing hybridoma obtained by the above described method include hybridomas GOT930-9 (FERM BP-6065), GOT935-54 (FERM BP-6066) and MOF3-139 (FERM BP-6059) (JP-A 10-155484, EP-A 834557) which produce monoclonal antibodies against surfactant compounds, as well as hybridomas MOF3-139 (FERM BP-6059), AP-14 (FERM BP-6633), BP2-177 (FERM BP-6634), DF-34 (FERM BP-6635) and CP-8 (FERM BP-6636) (PCT/JP99/00684, see Reference Example 3 hereinafter) which produce monoclonal antibodies against endocrine disruptors.

The production of the monoclonal antibody by the hybridoma and the purification thereof can also be carried out by a per se known method. The monoclonal antibody thus obtained can serve as an antibody against not only the environmental contaminants or their degradation products but also the compound of the formula (6).

Embodiments of production and purification of antibodies are described by, for example, the above ENZYME IMMUNOASSAY, pages 46-71 and 85-110, and can be carried out by salting-out (Na₂SO₄, (NH₄)₂SO₄), ion exchangers (DEAE, QAE, CM/cellulose, Sephadex, Sepharose, Servacel, etc.), hydrophobic chromatography (L-phenylalanyl-Sepharose, etc.), gel filtration (Sephadex G-200, Bio-Gel P-300, etc.), electrophoresis (zone electrophoresis with agarose gel, isoelectric electrophoresis, isokinetic electrophoresis, etc.), ultracentrifugation (sucrose density gradient centrifugation), affinity chromatography (immobilized protein A (Protein A Sepharose, Protein-A superose, etc.), and the like.

The above antibody can be bound to the above-obtained anit-Ig antibody supported on the carrier by using a per se known antigen-antibody reaction.

They can be combined with, for example, enzyme preparations for ELISA, buffering agents and the like to give a kit for immunoassay of environmental contaminants, degradation products thereof or a mixture thereof, or for immunological concentration of these materials.

In the immunoassay of the present invention, a so-called competitive assay is employed. That is, the quantitative determination can be carried out by quantitatively measuring competition between a specimen (e.g., water and soil samples containing environmental contaminants, their degradation products or a mixture thereof) and a known amount of a labeled environmental contaminant, its degradation product or a mixture thereof for binding to the monoclonal antibody of the present invention. In such competitive assay, a specimen solution containing an unknown antigen and a given amount of the antigen labeled with a labeling agent are added to a given amount of an antibody bound to an anti-Ig antibody supported on a carrier. The activity of the labeling agent supported by the carrier, or that of the labeling agent which is not supported by the carrier is measured. Desirably, the specimen and the labeled antigen are added almost at once.

In comparison with sandwich method or the like, the competitive assay can be carried out by a simpler and easier operation within a shorter period of time.

Examples of the agent for labeling the antigen include radioisotopes (hereinafter abbreviated to RI, e.g., ³H, ¹⁴C, ¹⁵N, ³²P, ¹³¹I, etc.), enzymes (e.g., peroxidase, β-galactosidase, alkaline phosphatase, urease, glucose oxidase, glucoamylase, carbonic anhydrase, acetylcholinesterase, lysozyme, malate dehydrogenase, glucose-6-phosphate dehydrogenase, ribonuclease, cytochrome C), enzyme substrates (e.g., 3,3',5,5'-tetramethylbenzidine (TMBZ), o-phenylenediamine (OPD), o-toluidine, hydrogen peroxide, methyl hydroperoxide, ethyl hydroperoxide, propyl hydroperoxide, urea hydrogen peroxide, p-nitrophenylbenzoic acid, catechol, resorcinol, hydroquinone, pyrogallol, o-diaminobenzene, guaiacol, benzidine, lactose, o-nitrophenyl-β-D-galactoside, p-nitrophenylphosphoric acid, β-D-glucose, urea, amphetamine, barbituric acid, benzodiazepine, benzoyl ecgonine), methadone, morphine, propoxyphene, L-malic acid, thyroxine, codeine, triiodothyronine, tetrahydrocannabinol, lidocaine, phencyclidine, theophylline, digoxin, cortisol, phenytoin, phenobarbital, primidone, benzodiazepam, carbamezepine, ethosuccimide, gentamicin, quinidine, procainamide, methotrexate), fluorescent materials (e.g., umbelliferone galactoside), luminous materials (e.g., luciferase), biotin and the like. For binding the labeling agent to the antigen, maleimide method [J. Biochem., 79, 233 (1976)], active biotin method [J. Am. Chem. Soc., 100, 3585 (1978)] and the like can be used.

For example, specific immunochemical determination by the competitive assay can be carried out by adding a solid phase, to which an antibody is bound, to a sample to be tested, which contains an unknown amount of a environmental contaminant, to allow to react. At the same time, a given amount of an antigen labeled with a labeling agent is added thereto to allow to react. Then, normally, the solid phase is washed thoroughly and the activity of the labeling agent bound to the solid phase is measured. When the labeling agent is RI, the measurement is carried out with a well counter or a liquid scintillation counter. When the labeling agent is an enzyme, its substrate is added, the mixture is allowed to stand and then the enzymatic activity is measured by colorimetry or fluorometry. When the labeling agent is a fluorescent material or a luminous material, it is measured according to a known method.

In the immunological determination or assay of the present invention, by using the complex of the anti-Ig antibody supported on the carrier and the mpnoclonal antibody against the environmental contaminant or its degradation product bound to the anti-Ig antibody as the solid phase, the determination or assay can be simply and readily carried out with high sensitivity.

The following Reference Examples and Examples further illustrate the present invention in detail but are not to be construed to limit the scope thereof.

The above hybridomas and the hybridoma of Reference Example 3 hereinafter have been deposited at National Institute of Bioscience and Human-Technology (NIBH), Agency of Industrial Science & Technology, Ministry of International Trade & Industry of 1-1-3 Higasi, Tsukuba-shi, Ibaraki-ken, Japan under Budapest Treaty. The accession number of each hybridoma and the date of deposition are listed in the following table.

**Table 1**

| Monoclonal antibody | Hybridoma | Accession number | Date of deposition |
|---|---|---|---|
| Anti-LAS antibody | GOT930-9 | FERM BP-6065 | Sept. 25/1996 |
| Anti-LAS antibody | GOT935-54 | FERM BP-6066 | Sept. 25/1996 |
| Anti-APE antibody | MOF3-139 | FERM BP-6059 | Aug. 13/1997 |
| Anti-PRC antibody | BP2-177 | FERM BP-6634 | Feb. 2, 1999 |
| Anti-AP antibody | AP-14 | FERM BP-6633 | Feb. 2, 1999 |
| Anti-PP antibody | DF-34 | FERM BP-6635 | Feb. 2, 1999 |
| Anti-CP antibody | CP-8 | FERM BP-6636 | Feb. 2, 1999 |

### Reference Example 1

### Preparation of solid phase anti-mouse IgG antibody supported on carrier

An anti-mouse IgG antibody [antigen: mouse IgG; purchased from ICN Pharmaceuticals, Inc. (Kappel Product), Cosmo Bio Co., Ltd.; catalogue No. 55479] was dissolved in Dulbecco's phosphate buffered saline (PBS) at a concentration of 50 µg/ml and the solution was added to a microplate (carrier) in an amount of 100 µl/well. After reacting at 4°C overnight, the microplate was washed with phosphate buffered saline containing 0.05% Tween 20 (TPBS) and Blockace (manufactured by Snow Brand Milk Products Co., Ltd., Tokyo) diluted 4 times with PBS was added thereto in an amount of 150 µl/well. After reacting at 37°C for more than 1 hour, the desired solid phase anti-mouse IgG antibody supported on the carrier was obtained and stored at 4°C until it was used.

### Reference Example 2

### Preparation of solid phase anti-mouse IgA antibody supported on carrier

An anti-mouse IgA antibody [antigen: mouse IgA; purchased from the same company as that of mouse IgG in Reference Example 1; catalogue No. 55478] was dissolved in Dulbecco's phosphate buffered saline (PBS) at a concentration of 50 µg/ml and the solution was added to a microplate (carrier) in an amount of 100 µl/well. After reacting at 4°C overnight, the microplate was washed with phosphate buffered saline containing 0.05% Tween 20 (TPBS) and Blockace (manufactured by Snow Brand Milk Products Co., Ltd., Tokyo) diluted 4 times with PBS was added thereto in an amount of 150 µl/well. After reacting at 37°C for more than 1 hour, the desired solid phase anti-mouse IgG antibody supported on the carrier was obtained and stored at 4°C until it was used.

### Reference Example 3

### Preparation of monoclonal antibody

### (1) Preparation of hapten for antibody against plastic resin components

Bisphenol A (25 g) and glutaric anhydride (12.5 g) were reacted at 100°C for 18 hours under a nitrogen atmosphere. The reaction mixture was dissolved in ethyl acetate and purified by a silica gel column and an octadecyl silica gel (ODS) column, followed by crystallization form hexane to obtain bisphenol-glutaric acid condensate (2.1 g).

### (2) Preparation of immunogen

The hapten obtained in (1) (0.1 mol), watersoluble carbodiimide (0.14 mol), N-hydroxysuccinimide (0.14 mol) were reacted in dimethylsulfoxide (2ml) at room temperature overnight to obtain an activated ester. Then, the activated ester (200 µl) was added to a solution of bovine serum albumin (BSA, 10 mg) in 0.13 M sodium bicarbonate (NaHCO₃) solution and the reaction was carried out at 4°C overnight. After removing unreacted reagents with ultrafiltration, the resultant was frozen and stored to be used as an immunogen.

### (3) Immunization

To a solution of the complex of the hapten and BSA obtained in (2) in physiological saline (500 µg/ml) was added the equal amount of Freund's complete adjuvant or RIBI adjuvant. After thoroughly emulsified, it was inoculated into BALB/c mice (female, 100 µg/mouse) subcutaneously and booster immunization was conducted 2 week intervals (Freund's adjuvant) or 3 week intervals (RIBI adjuvant). After immunizing booster 5 to 6 times, the same complex solution (5 to 10 µg/0.1 ml physiological saline/mouse) was administered to individuals which showed maximum serum antibody titers intravenously.

### (4) Cell fusion

Three days after the last immunization, the spleen was excised from the abdomen of each mouse and a suspension of spleen cells (about 10⁸ cells) was prepared according to a conventional manner. Then, mouse myeloma cells (P3U1) (2 x 10⁷ cells) which had been washed with a serum-free culture medium three times were added thereto and subjected to cell fusion with PEG 6000 according to the method disclosed by Koehler and Milstein [Nature, 256, 495 (1975)].

After completion of the fusion, the cell mixture was suspended in so-called HAT medium containing hypoxanthine, aminopterin and thymidine and incubated for 10 days. Cells originating from the spleen died out during the 10 days incubation period spontaneously and P3U1 strain which did not fused together with the spleen cells also died out due to HAT medium. The cells which survived in the medium were regarded as hybridomas and then incubation was continued by replacing the medium with HT medium corresponding to aminopterin-free HAT medium.

### (5) Primary selection and cloning of hybridomas

An antibody titer of the supernatant of the hybridoma culture was measured by ELISA using a microplate in which a complex of a hapten and OVA prepared by the same manner as that used for the immunogen (hereinafter abbreviated to hapten-OVA) was adsorbed on the solid phase. From 10 days to 20 days after fusion, the appearance of hybridomas were observed and the appearance of an antibody which linked to the hapten-OVA was recognized. Hybridomas which produced antibodies showing especially high binding activity were subjected to cloning by limiting dilution analysis.

### (6) Secondary selection of hybridomas

Regarding hybridomas obtained by the method of the above (5), a subject compound (plastic resin component, bisphenol A) was added to the supernatant of the hybridoma cultures and the hybridomas were subjected to binding inhibitory examination wherein inhibition of binding to a hapten-OVA-adsorbing microplate was examined and a hybridoma whose binding was inhibited only by the subject compound was selected.

As a result, a hybridoma (BP2-177) which produced a monoclonal antibody specifically binding to the subject compound was obtained.

### (7) Production of monoclonal antibody

The cloned hybridoma was cultivated by incubating it in Daigo T medium (Nippon Seiyaku, Tokyo, Japan) containing 10% bovine fetal serum at 37°C under 5% CO₂ and the antibody was obtained from the supernatant of the culture.

On the other hand, for obtaining a large amount of antibody, 0.5 ml of mineral oil was administered to a BALB/c mouse intraperitoneally and then 5 x 10⁶ cells of hybridoma were inoculated in the mouse intraperitoneally. After about 10 days, the collection of ascites fluid was observed in the mouse that received inoculation of the cells and the ascites fluid was harvested from the peritoneal cavity by abdominal section of the mouse.

The purification of the antibody was carried out by fractionating a fraction from the supernatant of cell culture or ascites fluid with 45 to 50% saturated ammonium sulfate according to a conventional method and subjecting to column chromatography on a protein A column. Thus, a monoclonal antibody against the plastic resin component, anti-PRC antibody, was obtained.

### (8) Preparation of other hybridomas

### 1) Preparation of hapten for antibodies against alkylphenolethoxylates (APE) and alkylphenols (AP)

Polyethyleneglycol monononylphenol ether (NP5EO) (5.0 g) was dissolved in toluene (60 ml) and reacted with succinic anhydride (918 mg) and p-toluenesulfonic acid (16 g) at 80°C for 2 hours under a nitrogen atmosphere. The reaction mixture was washed with ether under alkaline conditions (pH 12) and extracted with chloroform under acidic conditions (pH 2). The extract was dehydrated and evaporated to dryness to obtain the desired hapten (2.1 g).

### 2) Preparation of hapten for antibodies against plastic plastisizers (PP)

8-Bromooctanoic acid (10 g) was dissolved in tetrahydrofuran (300 ml) and to this was added diphenylaminomethane (20 ml), followed by reaction at room temperature overnight. On the next day, diphenylaminomethane (20 ml) was further added thereto and the mixture was further reacted at room temperature overnight. After concentration at reduced pressure, the reaction mixture was dissolved in hexane-ethyl acetate (9 : 1) and purified roughly on a silica gel column.

The resultant roughly purified product (20 g) together with phthalic acid (2.42 g) and 1,8-diazabicycloundecene (DBU) (2.22 g) in benzene (60 ml) were heated under reflux overnight. On the next day, DBU (2.22 g) was added thereto and the mixture was heated under reflux for additional 6 hours. After cooling to room temperature, water and chloroform were added and the mixture was washed with water. The chloroform layer was dehydrated, dissolved in hexane-ethyl acetate (9 : 1), and purified roughly on a silica gel column.

The resultant roughly purified product (4.1 g) was dissolved in tetrahydrofuran (THF) (100 ml) and to the solution was added 10% Pd/C (50% water containing product) (0.4 g). After bubbling H₂ (0.3 ml/min., for 5 hours), the catalyst was removed and the reaction mixture was concentrated at reduced pressure. The concentrate was dissolved in 75% methanol and purified with an ODS column to obtain the desired hapten (1.9 g).

### 3) Preparation of hapten for antibody against chlorophenols (CP)

5-Bromovaleric acid (50 g) and triphenylphosphin (72.5 g) in toluene (400 ml) were heated under reflux overnight and the reaction mixture was filtrated. The solids obtained were washed with toluene and dried at reduced pressure to obtain the phosphonium salt (120 g). Separately, p-hydroxybenzaldehyde (25 g) was dissolved in acetic acid (125 ml) (35°C) and warmed to 50°C. Chlorine gas was bubbled for 2 hours. The reaction mixture was allowed to cool to room temperature (35°C) to obtain white crystals (19 g). The crystals (19 g) were mixed with acetic acid (36 g) and the mixture was heated to 80°C, followed by bubbling chlorine gas to bring about thick slurry. After allowing to cool to room temperature, the precipitated white crystals were recrystallized from chloroform to obtain 3,5-dichloro-4-hydroxybenzaldehyde (9.3 g). Then, these crystals (9 g), the above phosphonium salt (31.3 g) and t-butanol (15.5 ml) were dissolved in toluene (210 ml) and, after heating to 50°C, t-BuOK (23.7 g) was added thereto. The mixture was reacted at 55°C for 5 hours and further at room temperature overnight. The reaction mixture was poured into ice, the toluene layer was discarded and the remaining solution was adjusted to pH 2. The solution was extracted with ethyl acetate and the extract was dried at reduced pressure. The residue was dissolved in hexane-ethyl acetate (9 : 1) and purified with a silica gel column to obtain a purified product (5.3 g). The purified product (2.55 g), 10% Pd/C (50% water containing product) (0.36 g), water (30 ml) and methanol (60 ml) were mixed and hydrogen was bubbled (0.2 L/min., for 20 minutes). After removing the catalyst, the reaction mixture was concentrated at reduced pressure and extracted with isopropyl ether (IPE). After concentration at reduced pressure, the extract was purified with a silica gel column and an ODS column. The purified product was recrystallized from ether/hexane to obtain the desired hapten (1.7 g).

According to the same manner as described in the above (2) to (6), hydridomas MOF3-139 (producing anti-APE antibody, FERM BP-6059), AP-14 (producing anti-AP antibody, FERM BP-6633), DF-34 (producing anti-PP antibody, FERM BP-6635) and CP-8 (producing anti-CP antibody, FERM BP-6636) were prepared by using these haptens.

### Example 1

### (1) Immobilization of anti-estrogen monoclonal antibody

After washing the solid phase anti-mouse IgG supported on the carrier prepared in Reference Example 1 with TPBS, to this was added 100 µl/well (0.0125 µg antibody/well) of anti-estrogen monoclonal antibody (anti-estriol 16-glucuronide monoclonal antibody purchased from Teikoku Hormone Mfg. Co., Ltd., Tokyo) which was diluted at an optimal concentration (0.125 µg antibody/ml) with PBS and reacted at 4°C overnight. After washing with PBS, 150 µl/well of Blockace diluted 4 times with TPBS was added. The mixture was reacted at 37°C for more than 1 hour to obtain a complex of carrier-anti-mouse IgG antibody-antiestrogen monoclonal antibody. This was stored at 4°C until it was used.

### (2) Determination of estradiol with anti-estrogen antibody

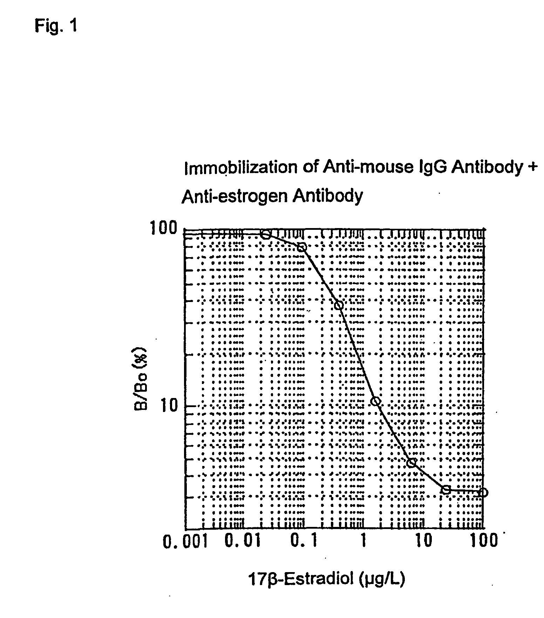

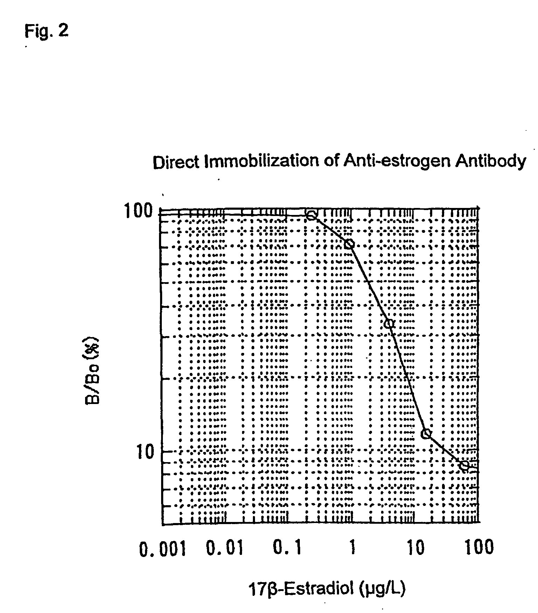

An antigen-enzyme complex (estriol 16 glucuronide was bound to horseradish peroxidase by carbodiimide method) was diluted with 2-fold concentrated PBS (containing 0.1% Tween 20) and it was mixed with the equal amount of a sample containing estrogen. The mixture was added to an assay plate. After reaction at room temperature for 60 minutes, the reaction mixture was washed with TPBS and a substrate for color development was added thereto. After reaction at room temperature for 30 minutes, the color development reaction was stopped with 1N phosphoric acid and the absorbance was measured to obtain the calibration curve as shown in Fig. 1. As a control, the same antibody was immobilized on the carrier directly (0.5 µg antibody/well) and, according to the same manner, the absorbance was measured to obtain the calibration curve as shown in Fig. 2.

These results show that the amount of anti-estrogen antibody used can be reduced to about 1/40 by immobilizing the anit-mouse IgG antibody and the sensitivity is improved about 10 times.

### Example 2

### (1) Immobilization of anti-plastic resin component (PRC) monoclonal antibody

After washing the solid phase anti-mouse IgA supported on the carrier prepared in Reference Example 2 with TPBS, to this was added 100 µl/well (0.03 µg antibody/well) of anti-PRC monoclonal antibody (prepared in Reference Example 3) which was diluted at an optimal concentration (0.3 µg/ml) with TPBS and reacted at 4°C overnight. After washing with PBS, 150 µl/well of Blockace diluted 4 times with PBS was added. The mixture was reacted at 37°C for more than 1 hour to obtain a complex of carrier-anti-mouse IgA antibody-anti-PRC monoclonal antibody. This was stored at 4°C until it was used.

### (2) Determination of bisphenol A (BPA) with anti-PRC antibody

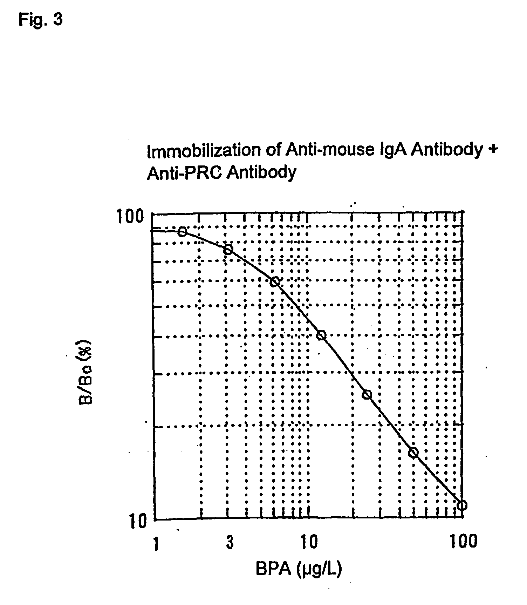

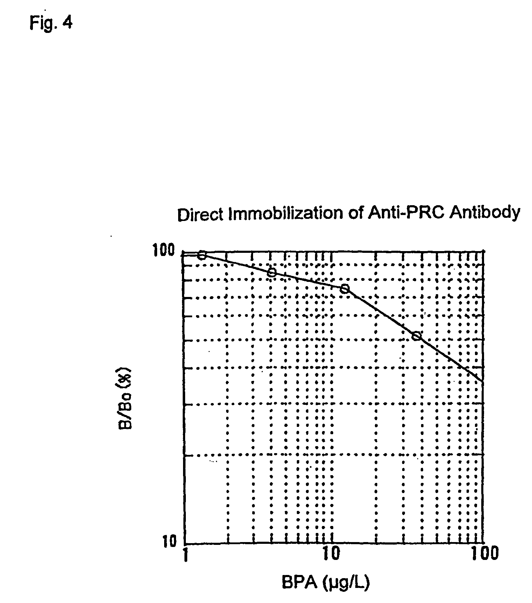

An antigen-enzyme complex (BPA and half ester of glutaric anhydride were boundto horseradish peroxidase by carbodiimide method) was diluted with 2-fold concentrated PBS and it was mixed with the equal amount of a sample containing BPA. The mixture was added to an assay plate. After reaction at room temperature for 60 minutes, the reaction mixture was washed with TPBS and a substrate for color development was added thereto. After reaction at room temperature for 30 minutes, the color development reaction was stopped with 1N phosphoric acid and the absorbance was measured to obtain the calibration curve as shown in Fig. 3. As a control, the same antibody was immobilized on the carrier directly (0.5 µg antibody/well) and, according to the same manner, the absorbance was measured to obtain the calibration curve as shown in Fig. 4.

These results show that the amount of anti-PRC antibody used can be reduced to about 1/17 by immobilizing the anit-mouse IgA antibody and the sensitivity is improved about 5 times.

As described hereinabove, an environmental contaminant and its degradation product can be simply and readily determined with high sensitivity and specificity by immunoassay using the kit of the present invention whose essential constituent component is a complex of an anti-Ig antibody supported on a carrier and a further antibody against the environmental contaminant or its degradation product bound to said anti-immunoglobulin antibody.

## Claims

1. A kit for immunoassay of an environmental contaminant, its degradation product or a mixture thereof which comprises as an essential constitutional component a complex of an anti-immunoglobulin antibody supported on a carrier and a further antibody against the environmental contaminant or its degradation product bound to said anti-immunoglobulin antibody.

2. The kit according to claim 1, wherein the environmental contaminant is a surfactant compound for synthetic detergents or an endocrine disruptor.

3. The kit according to claim 2, wherein the surfactant compound is a compound selected from the group consisting of anionic surfactant compounds and nonionic surfactant compounds.

4. The kit according to claim 2, wherein the surfactant compound is a compound selected from the group consisting of C₂₋₁₈ alkyl sulfate, C₂₋₁₈ alkylbenzene sulfonate, C₂₋₁₈ alkylnaphthalene sulfonate, naphthalene sulfonate-formaldehyde condensate, polyoxyethylene C₂₋₁₈ alkylphenyl ether, polyoxyethylene C₂₋₁₈ alkyl ether, polyoxyether polystyryl phenyl ether, polyoxyethylene styrylated phenyl ether and salts thereof.

5. The kit according to claim 2, wherein the endocrine disruptor is a substance selected from the group consisting of female sex hormones, male sex hormones, thyroid hormones, alkylphenol ethoxylates, alkylphenols, plastic resin components, plasticizers and chlorophenols.

6. The kit according to claim 2, wherein the endocrine disruptor is a substance selected from the group consisting of estrogen, estradiol, estrone, estriol, androgen, testosterone, dehydroepiandrosterone, androstenedione, thyroxine, triiodothyronine, C₁₋₂₀ alkylphenol ethoxylates, C₁₋₂₀ alkylphenols, bisphenol A, 4,4'-ethylidene bisphenol, bis(p-hydorxyphenyl)methane, 2,2'-bis(4-hydroxypehyl)-1-propanol, 2,2'-bis(m-methyl-p-hydroxyphenyl)propanel, 4,4'-bis(p-hydroxyphenyl)pentanoic acid, 4,4'-diaminodiphenylmethane, 4,4'-dihydroxydiphenyl ether, 4,4'-dihydroxydiphenylsulfone, 4,4'-dichlorodiphenylsulfone, bis[4-(2-hydroxyethoxy)-3,5-dibromophenyl]sulfone, bis[4-(2-hydroxyethoxy)phenyl]sulfone, p,p'-dihydorxybenzophenone, 4-hydroxybiphenol, butyl benzyl phthalate, dibutyl phthalate, dicyclohexyl phthalate, diethyl phthalate, di(2-ethylhexyl) phthalate, diethyl hexyl adipate, dihexyl phthalate, di-n-pentyl phthalate, dipropyl phthalate, and mono-, di-, tri-, tetra- and penta-chlorophenols.

7. The kit according to claim 1, wherein the anti-immunoglobulin antibody is anti-IgG antibody or a antibody mixture containing it.

8. A method for immunoassay of an environmental contaminant, its degradation product or a mixture thereof which comprises:
reacting (a) a specimen, (b) a complex of an anti-immunoglobulin antibody supported on a carrier and a further antibody against the environmental contaminant or its degradation product bound to said anti-immunoglobulin antibody, and (c) the environmental contaminant, its degradation product or a mixture thereof labeled with a labeling agent, and
measuring the activity of either the labeling agent held on the carrier or the labeling agent not held on the carrier.

9. The method according to claim 8, wherein the environmental contaminant is a surfactant compound for synthetic detergents or an endocrine disruptor.

10. The method according to claim 9, wherein the surfactant compound is a compound selected from the group consisting of anionic surfactant compounds and nonionic surfactant compounds.

11. The method according to claim 9, wherein the surfactant compound is a compound selected from the group consisting of C₂₋₁₈ alkyl sulfate, C₂₋₁₈ alkylbenzene sulfonate, C₂₋₁₈ alkylnaphthalene sulfonate, naphthalene sulfonate-formaldehyde condensate, polyoxyethylene C₂₋₁₈ alkylphenyl ether, polyoxyethylene C₂₋₁₈ alkyl ether, polyoxyether polystyryl phenyl ether, polyoxyethylene styrylated phenyl ether and salts thereof.

12. The method according to claim 9, wherein the endocrine disruptor is a substance selected from the group consisting of female sex hormones, male sex hormones, thyroid hormones, alkylphenol ethoxylates, alkylphenols, plastic resin components, plasticizers and chlorophenols.

13. The method according to claim 9, wherein the endocrine disruptor is a substance selected from the group consisting of estrogen, estradiol, estrone, estriol, androgen, testosterone, dehydroepiandrosterone, androstenedione, thyroxine, triiodothyronine, C₁₋₂₀ alkylphenol ethoxylates, C₁₋₂₀ alkylphenols, bisphenol A, 4,4'-ethylidene bisphenol, bis(p-hydorxyphenyl)methane, 2,2'-bis(4-hydroxypehyl)-1-propanol, 2,2'-bis(m-methyl-p-hydroxyphenyl)propanel, 4,4'-bis(p-hydroxyphenyl)pentanoic acid, 4,4'-diaminodiphenylmethane, 4,4'-dihydroxydiphenyl ether, 4,4'-dihydroxydiphenylsulfone, 4,4'-dichlorodiphenylsulfone, bis[4-(2-hydroxyethoxy)-3,5-diburomophenyl]sulfone, bis[4-(2-hydroxyethoxy)phenyl]sulfone, p,p'-dihydorxybenzophenone, 4-hydroxybiphenol, butyl benzyl phthalate, dibutyl phthalate, dicyclohexyl phthalate, diethyl phthalate, di(2-ethylhexyl) phthalate, diethyl hexyl adipate, dihexyl phthalate, di-n-pentyl phthalate, dipropyl phthalate, and mono-, di-, tri-, tetra- and penta-chlorophenols.

14. The method according to claim 8, wherein the anti-immunoglobulin antibody is anti-IgG antibody or a antibody mixture containing it.

15. Use of a complex of an anti-immunoglobulin antibody supported on a carrier and a further antibody against the environmental contaminant or its degradation product bound to said anti-immunoglobulin antibody in immunoassay of an environmental contaminant, its degradation product or a mixture thereof.

16. Use according to claim 15, wherein the anti-immunoglobulin antibody is anti-IgG antibody or a antibody mixture containing it.
